# EUROPEAN PATENT APPLICATION

(11) **EP 4 501 234 A1**
(43) Date of publication of application: **05.02.2025**
(21) Application number: 23306321.3
(22) Date of filing: 01.08.2023
(51) Int. Cl.: A61B 5/291, A61B 5/31, A61B 5/256, A61B 5/16, A61B 5/374

(54) **DEVICE FOR DETERMINING A PHYSIOLOGICAL OR PSYCHOLOGICAL STATE OF A MAMMAL AND ASSOCIATED METHOD**

(71) Applicant: NAOX Technologies, 91120 Palaiseau (FR)
(72) Inventor: LANGE, Emmanuel, 91120 Palaiseau (FR); DINH, Hugo, 91120 Palaiseau (FR); KABABE, Khalil, 91120 Palaiseau (FR); VALDERRAMA, Mario, 91120 Palaiseau (FR)
(74) Representative: Icosa

(57) **Abstract**

The present invention relates to a device for determining a physiological or psychological state of a mammal, the device comprising at least one earpiece comprising:
- a main body (3) comprising an elongation body extending along an elongation axis, and
- an earmold (4) configured to be inserted into an ear canal of the mammal and comprising at least one electrode arranged on an outer surface of the earmold (4), the at least one electrode being configured to detect at least one electrical signal in the ear canal of the mammal, the earmold being configured to convey the at least one electrical signal to a processing unit,
wherein the processing unit comprise at least one preamplifier positioned upstream in the direction of propagation of the at least one electrical signal.

## Description

### FIELD OF INVENTION

The present invention relates to the field of devices for determining a physiological or psychological state of a mammal, in particular devices for determining such a state by measuring an electroencephalogram (EEG) signal.

### BACKGROUND OF INVENTION

An in-ear device for measuring biological data of a human, such as the one described by US 2018/0235540, is known, which comprises an interchangeable portion intended to be inserted into an ear canal provided on its outer surface with electrodes intended to detect an electrical signal from the heart.

The detection of electrical signals by the electrodes may lack accuracy. Indeed, such an in-ear device is sensitive to external and internal noises.

The invention aims at providing a solution in this regard and, more particularly, at overcoming the aforementioned disadvantages and at providing a biological data measuring device capable of providing more accurate signals, and having a simple, inexpensive and compact structure.

### SUMMARY

This invention thus relates to a device for determining a physiological or psychological state of a mammal, the device comprising at least one earpiece comprising:
- a main body comprising an elongation body extending along an elongation axis, and
- an earmold configured to be inserted into an ear canal of the mammal, the earmold having a channel adapted to receive said elongation body to removably mount the earmold onto the main body, said earmold comprising at least one electrode arranged on an outer surface of the earmold, said at least one electrode being configured to detect at least one electrical signal in the ear canal of the mammal, the earmold being arranged to be rotatable about the elongation axis so as to allow said at least one electrode to be directed to an area of the brain of said mammal;

the earpiece comprising at least one first electrical track arranged in the channel of the earmold and connected to the at least one electrode, and at least one second electrical track arranged in the elongation body of the main body and configured to convey the at least one electrical signal detected by said at least one electrode to processing unit, wherein said processing unit comprise at least one preamplifier positioned upstream in the direction of propagation of said at least one electrical signal detected by said at least one electrode, said at least one electrical signal having a given intensity,
the at least one preamplifier being configured to amplify the given intensity of at least one electrical signal detected by the at least one electrode.

Thus, the at least one preamplifier allows amplifying the intensity of the electrical signals detected by the at least one electrode of the device, making the electrical signals more prominent with respect to external and internal noises also measured by the device when the device is in use.

In some embodiments, the at least one preamplifier is housed in the elongation body.

In some embodiments, the at least one preamplifier presents a working frequency range extending from 0.1 Hz to 1 kHz.

Thus, this position of the at least one preamplifier allows amplifying the electrical signals detected by the at least one electrode close to the location where those electrical signals are captured, therefore minimizing the capturing of unwanted signals.

In some embodiments, each among the at least one preamplifier is coupled to an ESD (electrostatic discharge) circuit. Therefore, advantageously, the ESD circuit protects the device according to the invention from electrostatic discharges that can occur for instance when a user of the device touches the device.

In some embodiments, the at least one preamplifier comprises a follower circuit. A follower circuit is also referred to as buffer or unity gain amplifier and is based on an operational amplifier. Advantageously, the output provided by the operational amplifier is identical to the input in terms of voltage, but with a higher current capability. Therefore, advantageously, the follower circuit provide a low output impedance to minimize signal losses when the follower circuit is connected to a load.

In some embodiments, the at least one preamplifier comprises an impedance matching circuit. Such an impedance matching circuit modifies the impedance of an electrical signal source, here, the body of the mammal, to match the impedance of a connected load (here, processing unit that will be further described). In this way, the impedance matching circuit optimizes the power transfer between the body of the mammal and the load by minimizing losses due to impedance mismatch. Typically, when the mammal is a human person, the input impedance lies in the Tera-ohms (TΩ) range, whereas, at the output of the impedance matching circuit, the impedance lies in the kilohms (kΩ) range.

In some embodiments, the at least one preamplifier is mounted on a printed circuit board of maximum dimension 3 millimeters.

In some embodiments, the printed circuit board is made out of a flexible material, so as to be folded. In this manner, the at least one preamplifier can be integrated for instance inside the elongation body of the main body in an easy and compact manner.

For instance, the flexible material can be chosen among polyimides such as Kapton, polyetheretherketone (PEEK), polymers, PTFE (Teflon).

In some embodiments, the printed circuit board comprises a U-shape so as to embrace additional components embedded in the elongation body. In this manner also, the at least one preamplifier can be integrated for instance inside the elongation body of the main body in an easy and compact manner.

In some embodiments, the earpiece is further configured to transmit a sound signal into the ear canal by means of at least one electroacoustic transducer housed in the elongation body, the at least one preamplifier being attached to the at least one electroacoustic transducer along the circumferential direction of the elongation axis. Therefore, the device according to the invention can be multifunctional while remaining compact.

In some embodiments the elongation body of the main body embeds a signaling LED and the at least one preamplifier is mounted on the signaling LED.

In some embodiments referred to as single earpiece embodiments, the at least one electrode comprises at least a first electrode being configured to detect a first electrical signal and a second electrode configured to detect a second electrical signal,
the at least first electrical track comprises at least two first electrical tracks, and the at least second electrical track comprises at least two second electrical tracks,
the first electrode and second electrode are spaced apart in a circumferential direction about the elongation axis,
the at least two first electrical tracks are electrically insulated from each other,
the at least two second electrical tracks being electrically insulated from each other,
the processing unit are connected to at least one processor,
the at least one processor being configured to determine the physiological or psychological state as a function of said first electrical signal measured by the first electrode and of the second electrical signal measured by the second electrode, in particular as a function of a difference between the first electrical signal and the second electrical signal. Those single earpiece embodiments are based on the use of a device using only one earpiece.

In other embodiments referred to as dual earpiece embodiments, the device comprises two earpieces configured to be arranged respectively in a first ear canal of the mammal and a second ear canal of the mammal and configured to convey respective electrical signals to the processing unit,
the processing unit being connected to at least one processor,
the at least one processor being configured to determine the physiological or psychological state as a function of a signal measured by an electrode of the earpiece hosted in the first ear canal and a signal measured by an electrode of the earpiece hosted in the second ear canal, in particular as a function of a difference between the two signals. Those dual earpiece embodiments are based on the use of a device using two earpieces.

Another aspect of the invention relates to computer-implemented method for determining a physiological or psychological state of a mammal by means of at least one processor of the device previously described, the method comprising:
- determining an electroencephalogram signal of the mammal based on the detected electrical signals,
- determining an amplitude of at least one brain wave in a predefined frequency range as a function of said electroencephalogram signal, and
- determining a psychic state based on said amplitude of at least one brain wave by comparing said amplitude to a predetermined threshold.

In some embodiments, the method further comprises a step of computing a quality index of the electrical signals detected by the electrodes based on a correlation computation between a common mode measurement and said difference between said first electrical signal and said second electrical signal or said difference between said two signals.

### DEFINITIONS

In the present invention, the following terms have the following meanings:

The terms **"adapted"** and **"configured"** are used in the present disclosure as broadly encompassing initial configuration, later adaptation or complementation of the present device, or any combination thereof alike, whether effected through material or software means (including firmware). The term **"reference electrode"** refers to an electrode used in EEG to establish a baseline voltage or reference point for measuring the electrical potentials recorded by the measurement electrodes. It serves as a point of comparison against which the electrical activity from other electrodes is measured. The reference electrode does not directly measure brain activity; instead, it provides a fixed voltage level that helps in interpreting the electrical signals from other electrodes.

The term **"measurement electrode",** also known as a measure electrode or recording electrode, refers to an electrode configured to directly measure the electrical activity generated by the brain. It detects the voltage fluctuations resulting from the electrical signals produced by neural activity in the brain. The measure electrodes are the primary electrodes used for capturing EEG signals.

### BRIEF DESCRIPTION OF THE DRAWINGS

**Figure 1** represents a side view of a device for determining a physiological or psychological state of a mammal according to some embodiments.
**Figure 2** represents an inside view of the device of Figure 1 in the plane of Figure 1.
**Figures 3A and 3B** are three-dimensional perspective views of a printed circuit board embedding a preamplifier used in the device of Figures 1 and 2, in an unfolded configuration (**3A**) and in a folded configuration while embedded (3B) in a channel, according to some embodiments.
**Figure 4** is a schematic figure of processing unit configured to be used in a device as represented in Figures 1 and 2.
**Figure 5** represents a device for determining a physiological or psychological state of a mammal according to some embodiments different from Figures 1 and 2.
**Figure 6** represents an example of a flow chart representing steps for implementing a method for determining a physiological or psychological state of a mammal with a device such as illustrated on Figures 1, 2 and 5.

### DETAILED DESCRIPTION

This invention relates to a device 1a, 1b for determining a physiological or psychological state of a mammal, the device comprising at least one earpiece 2, 2' and to a computer-implemented method 100 for determining a physiological or psychological state of the mammal by means of at least one processor of the device 1. Preferably, the mammal is a person.

One single earpiece 2 configured to be inserted into an ear canal of the mammal and comprising at least two measurement electrodes can be used. Alternatively, two earpieces 2, 2', configured to be inserted into each ear canal of the mammal, each earpiece comprising at least one measurement electrode, can be used, both earpieces 2, 2' being possibly identical or different from each other. It will be further explained and described how the computer-implemented method 100 relies on the use of at least two measurement electrodes, both measurement electrodes being comprised into either one earpiece 2, or, each of the two electrodes being comprised into one among two separate earpieces 2, 2'.

More specifically, the device 1a, 1b is configured for determining electroencephalogram (EEG) data of a person.

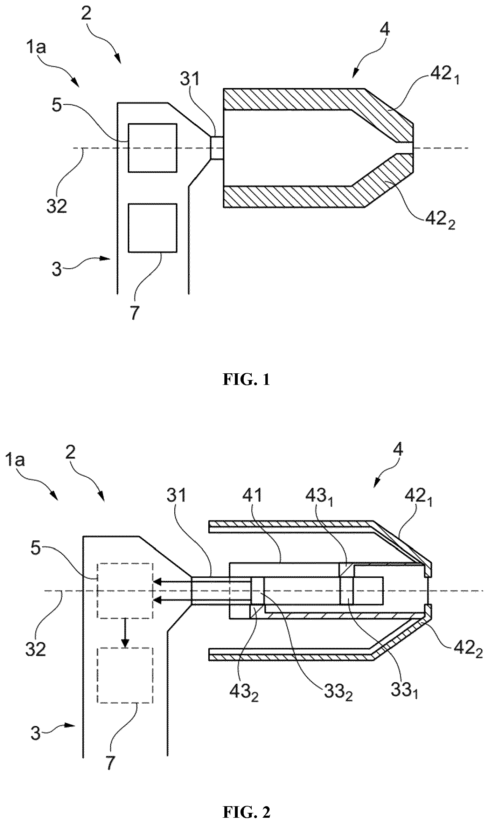

The earpiece 2, 2' will now be described and Figure 1 illustrates an example of the earpiece 2, 2'. For example, the earpiece 2, 2' is shaped and sized like an earphone, and includes a main body 3 and a removable earmold 4.

The main body 3 comprises an elongation body 31 extending along elongation axis 32. In one example, the elongation body 31 may be a body of revolution, wherein the elongation axis is an axis of revolution.

The removable earmold 4 is configured to be inserted into an ear canal of the person. The earmold 4 comprises a channel 41 adapted to receive the elongation body 21 of the main body 3 to removably mount the earmold 4 onto the main body 3. The earmold 4 comprises an outer surface with a plurality of N electrodes (42₁, 42ₖ.. 42_{N}) arranged on the outer surface of the earmold 4. For instance, the electrodes (42₁, 42ₖ.. 42_{N}) extend along the entire length of the earmold 4 in the direction of the elongation axis. For instance, the electrodes are spaced apart in a circumferential direction about the elongation axis 32. Other arrangements of the electrodes could be possible. On Figure 1, two electrodes 42₁ and 42₂ are visible.

Each electrode 42ₖ is configured to detect an electrical signal in the ear canal of the person. The earmold can arranged to be rotatable about the elongation axis 32 so as to allow at least one of the electrodes 42ₖ to be directed to an area of the brain of the person. For instance, the earmold 4 is made of insulating material, e.g., silicone, and the electrodes 42ₖ are, for example, pieces of conductive fabric embedded in the silicone of the earmold 4, e.g., by gluing. The earmold 4 is sized so as to ensure contact between the wall of the ear canal of the person and the electrodes 42ₖ.

As illustrated on Figure 2, the earpiece 2, 2' comprises first electrical tracks 43ₖ electrically insulated from each other arranged in the channel 41 of the earmold 4 and connected to the electrodes 42ₖ. For instance, the first electrical tracks 43ₖ can be contact pins connected to the electrodes 42ₖ by means of electrically insulated electrical wires, the contact pins being embedded on an inner surface of the channel 41 of the earmold 4.

The main body 3 includes a processing unit 5 configured to receive the electrical signals picked up by the electrodes 42ₖ of the earmold 4, process these signals and transform them into digital signals. Accordingly, the earpiece 2, 2' comprises second electrical tracks 33ₖ electrically insulated from each other arranged in the elongation body 31 of the main body 3 configured to convey the electrical signals detected by the electrodes 42ₖ to the processing unit 5. For instance, as illustrated on Figure 2, the second electrical tracks 33ₖ are annular electrical tracks having the elongation axis 32 as their axis and arranged at a distance from each other in the direction of the elongation axis 32. Each of the annular tracks is connected to an electrode 42ₖ of the earmold 4 by one corresponding first electrical track 43_{k.} The contact pins can be arranged opposite to the annular electrical tracks so as to maintain an electrical contact.

In some embodiments, the elongation body 31 is made of insulating material and the second electrical tracks 33ₖ are formed by a metal deposit on the elongation body 31. The second electrical tracks 33ₖ are connected to the processing unit5 by electrical wires arranged within the elongation body 31.

The processing unit 5 include at least one preamplifier 6, also referred to as buffer, positioned upstream in the direction of propagation of the electrical signals detected by the electrodes 42ₖ. There are N preamplifiers 6, each among them configured to receive a corresponding electrical signal from one among the N electrodes 42ₖ.

Each among the N preamplifiers 6 is configured to amplify the intensity of the received corresponding electrical signal. More specifically, each among the N preamplifiers 6 is configured to perform impedance matching. In other words, the impedance at the input of one preamplifier 6 is higher than the impedance at the output of this preamplifier. For instance, each among the N preamplifiers 6 comprises a follower circuit based on a differential amplifier.

Advantageously, the intensity gain of each among the N preamplifiers 6 is adjustable, therefore allowing versability in the distribution of intensity gains allowed by the N preamplifiers. In other words, the intensity of some electrical signals among the electrical signals detected by the electrodes 42ₖ may be amplified in a stronger manner than the intensity of the other electrical signals, so as to emphasize differential measurements between the electrical signal with strongly amplified intensity (i.e. signal of the neural activity obtained from an electrode working as measurement electrode) and an electrical signal with lower intensity from another electrode (i.e., baseline voltage obtained from an electrode working as reference electrode). As will be explained below, the device 1a, 1b is configured to perform and process such differential measurements. The adjustability of the intensity gain in an independent way of one or more of N preamplifiers 6 thus advantageously allows increasing the signal to noise ratios of the different detected electrical neural signals. In other words, as the neural recordings are performed using differential measurements, the possibility of strongly amplify the electrical signal(s) of the measurement electrodes using these N preamplifiers 6 increase the noise immunity of the device 1a, 1b and allows to recover a neural signal with an improved dynamic, i.e., a neural signal carrying more information. Meanwhile, the N preamplifiers 6 render the intensity of the electrical signals detected by the electrodes 42ₖ adjustable, therefore allowing possibly reducing the power consumption of the device 1a, 1b. This embodiment allowing to directly amplify analogically the desired electrical signal(s) provides better signal-to-noise ratio, while also reducing the energy consumption, with respect to other alternative methods which improves the difference between the baseline voltage and the measurement signal by numerically applying weighting factors to the signals during signal processing.

The presence of the preamplifiers 6 in the processing unit is also advantageous for the following reasons. The electrical current of the electrical signals corresponding to biological signals from the person and detected by the electrodes 42ₖ is of the order of magnitude of picoamps, which is comparable to the order of magnitude of external noises generated by external induction loops (with for example the main power supply) and internal noises also detected by the electrodes and. By internal noises, it is meant noises created by sources of noise present in the body of the mammal. Such sources of noise are for instance generated from the contraction of the facial muscles, eyelids or eyes movements, etc.

Therefore, the preamplifiers 6 allow preserving the input voltage of the electrical signals detected by the electrodes 42ₖ while increasing the electrical current. Typically, the impedance at the input of the preamplifiers 6 roughly corresponds to the impedance of the mammal skin and lies in the mega-ohm range, while the impedance at the output of the preamplifiers 6 lies in the kilohm range. Indeed, the brain of the mammal can be modeled as a voltage generator with an internal impedance. The internal impedance comprises the resistance of the head of the mammal and the resistance of the skin of the mammal.

Another advantage is the proximity of the preamplifiers 6 to the second electrical tracks 33ₖ, since the preamplifiers 6 are located in the elongation body 31 of the main body 3. This allows to amplify the corresponding electrical signals close to the location where the electrical signals of interest are captured and as a consequence to minimize the capturing of unwanted signals.

Besides, the presence of the intensity gain tunable preamplifiers 6 alleviates the need for downstream digital amplification, which contributes to power consumption reduction.

In some embodiments, each of the N preamplifiers 6 is coupled to an ESD protection circuit.

In some preferred embodiments, the size of each of the N preamplifiers 6 is configured so that the earpiece 2, 2' can be used when the person is a child. For instance, the size of the preamplifier/earpiece is smaller than a few millimeters, for instance smaller than 3 mm.

Advantageously, the N preamplifiers 6 are mounted on a printed circuit board 61.

In some preferred embodiments, the printed circuit board 61 is flexible, so as to be folded. For instance, the printed circuit board can made of a polyimide such as Kapton, polyetheretherketone (PEEK), polymers, PTFE (Teflon).

In alternative embodiments, the printed circuit board presents a U-shape or C-shape, or may be fold into a U-shape or a C-shape, so as to embrace other components embedded in the elongation body 31 of the main body 3.

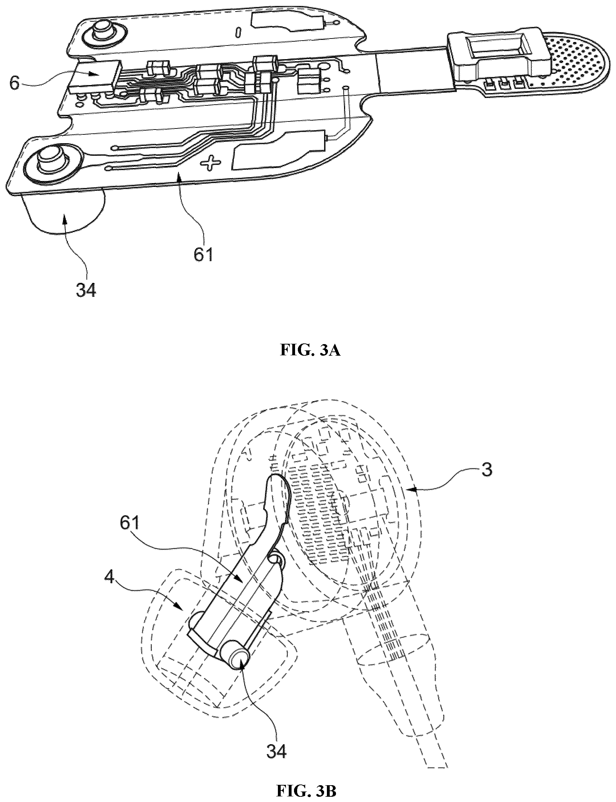

Figures 3A and 3B are examples of three-dimensional perspective views of one among the N preamplifiers 6 mounted on a printed circuit board. An electrode 34 is visible on Figure 3A, which is meant to be connected to one of the first tracks 43ₖ of the earmold 4. Figure 3B shows an example of the printed circuit board 61 on which one among the N preamplifiers 6 is mounted, and in a folded configuration so as to be integrated within the device 1. The corresponding electrode 34 is visible, as well as the earmold 4. As the earmold 4 is made out of a flexible material, it is pressed when the device 1 is inserted into one ear canal of the mammal for use. The distance between the ear canal surface and the end of the electrode 34, in this use configuration, is less than 1.5 mm.

For instance, in some embodiments, the earpiece 2, 2' is further configured to transmit a sound signal into the ear canal by means of at least one electroacoustic transducer housed in the elongation body 31 of the main body 3. In those embodiments, the printed circuit board in U-shape or C-shape, on which the preamplifiers 6 are mounted, can be positioned around said at least one electroacoustic transducer and, optionally, attached to the at least one electroacoustic transducer, for instance by gluing.

In other embodiments, the earpiece 2, 2' can comprise an additional functional component that can be positioned within the elongation body 31 of the main body 3, such as a signaling LED. Similarly, in those embodiments, the printed circuit board on which the preamplifiers 6 are mounted can be attached to the additional functional component.

In this manner, the inclusion of the preamplifiers 6 in the earpiece 2, 2' can be carried out simply while keeping the earpiece 2, 2'compact in size. Other arrangements of the preamplifiers 6 inside the elongation body 31 are possible. The positioning of the printed circuit board (i.e., the preamplifiers 6) in the elongation body 31 is advantageous as it allows to bring the amplifiers 6 closer to the electrodes 42ₖ so as to reduce the parasite noise.

The processing unit 5 is configured to output processed signals by digitizing and processing the electrical signals received by the electrodes 42ₖ and that have passed through the preamplifiers 6, towards at least one processor 7 that will be described further and that are configured to implement the computer-implemented method 100 for determining a physiological or psychological state of the mammal.

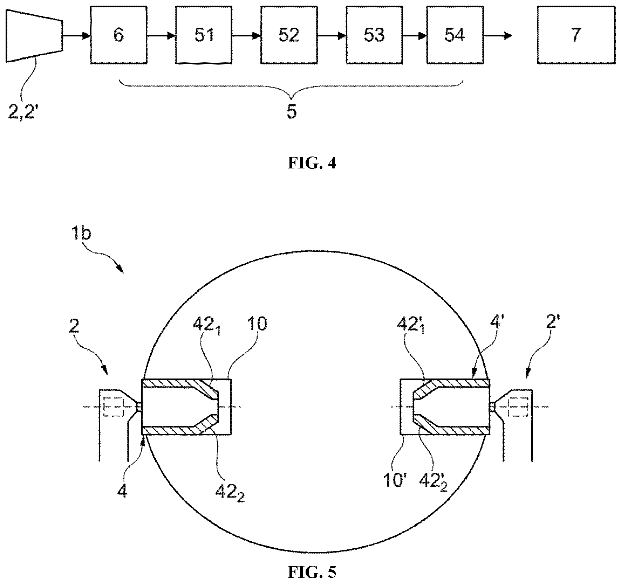

An example of chain of components comprised in the processing unit 5 including the preamplifiers 6 and other components downstream of the preamplifiers 6 is illustrated on Figure 4 and will be described below. It has to be noted that the preamplifiers 6 present, as explained above, a specific and independent technical function, and associated advantages, among the chain of components comprised in the processing unit 5.

Advantageously, the processing unit 5 is manufactured in a compact manner and can be encompassed in a limited volume. For instance, when the processing unit 5 is embedded inside the main body 3, it can be encompassed inside a cylinder of radius 6 mm and thickness 1 mm, leading to an encompassing volume of approximately 200 mm³.

First, an amplifier 51 is arranged to amplify the amplitude of the or each electrical signal. The gain of the amplifier 51 may be 10000 times in a single or multiple stages. The amplifier 51 is connected to an analog-to-digital converter (ADC) 52 configured to digitize the or each electrical signal. The ADC 52 is connected to a digital signal processing module 53 configured to attenuate any spurious signals picked up by the electrodes. These spurious signals may be due to the movement of the person's head, a movement of the electrical wires connected to the device 1, or the environment of the device 1. This digital signal processing module 53 includes in particular a bandpass filter, but also signal processing functions, in particular a function allowing a low frequency (or constant) component of the signal to be removed. This low frequency (or constant) component is present due to the fact of choosing a reference electrode inside the ear canal without using a mass located outside the ear canal. The bandwidth of the bandpass filter is further configured to select electrical signals from a predetermined organ, in particular the brain. For example, the bandwidth of the bandpass filter is between 0.5 Hz and 60 Hz, in particular between 1 Hz and 40 Hz. The bandpass filter is connected to a communication module 54 configured to transmit the signals digitized by the ADC 52 and filtered by the bandpass filter to the processor 7, in a wired or wireless manner.

The processor 7 comprises at least one processor. The processor 7 may be integrated into the main body 3 or separate therefrom, to determine EEG data based on the processed signals received from the processing unit 5.

In other embodiments, the processing unit 5 may also be arranged outside the main body. In those embodiments, the device 1a, 1b may comprise an antenna in the main body 3 or in the elongation body 31 downstream of the at least one preamplifier 6 so as to transmit the electrical signals captured by the electrodes to the processing unit 5.

As mentioned before, the device 1a, 1b may comprise one earpiece 2 (the device is then referred to as 1a) or two earpieces 2, 2' (the device is then referred to as 1b).

In so-called single earpiece embodiments, where the device is referred to as device 1a, one earpiece 2 configured to be inserted into an ear canal of the mammal and comprising at least two electrodes is used. The earmold 4 of the earpiece 2 comprises at least a first electrode 42i and a second electrode 42₂ comprised among the electrodes 42ₖ, each among the first electrode 42i and the second electrode 42₂ being configured to detect respectively a first electrical signal and a second electrical signal. In those embodiments, the at least one processor of the processor 7 is configured to determine a neural signal as a function of the first electrical signal and of the second electrical signal, in particular as a function of a difference between the first electrical signal and the second electrical signal. More specifically, one among the first electrode 42i and the second electrode 42₂ is chosen as the reference electrode, relative to which the potential will be calculated and the other electrode is therefore chosen as measurement electrode. The brain potential is calculated by differentiating the signals picked up by the other electrode from the signal picked up by the reference electrode. Preferably, the first electrode 42i and the second electrode 42₂ are as identical as possible.

In alternative so-called dual earpiece embodiments, where the device is referred to as device 1b, two earpieces 2, 2', configured to be inserted into each ear canal of the mammal are used, as represented on Figure 5. Each earpiece 2, 2' comprises at least one measurement electrode. In those embodiments, the at least one processor of the processor 7 is configured to determine the physiological or psychological state as a function of a signal measured by an electrode of the earpiece 2 hosted in the first ear canal and a signal measured by an electrode of the earpiece 2' hosted in the second ear canal, in particular as a function of a difference between both signals. Similarily to the single earpiece embodiments, one electrode of one earpiece 2, 2' is chosen as the reference electrode (while the other electrode will act as the measurement electrode), relative to which the potential will be calculated. The brain potential is calculated by differentiating the signals picked up by one electrode of the other earpiece from the signal picked up by the reference electrode.

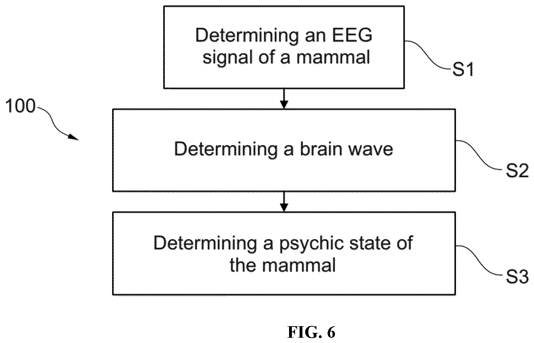

Another aspect of the invention relates to the computer-implemented method 100 for determining a physiological or psychological state of the mammal. The computer-implemented method 100 can be implemented by the processor 7 of the device 1a, 1b previously described. Figure 6 represents an example of steps that may be perform to implement the method 100.

In a step S1, the at least one processor determines an electroencephalogram signal of the mammal based on the detected electrical signals. As previously evoked in the description of the device 1a, 1b, the at least one processor computes the brain potential based on the difference between the signals of two electrodes, either two electrodes from one single earpiece 2, or one electrode of one earpiece 2 and another electrode of another earpiece 2'.

In a step S2, the at least one processor determines an amplitude of at least one brain wave in a predefined frequency range as a function of the electroencephalogram signal. For instance, a Fourier transform can be applied to determine the frequencies contained in the electroencephalogram signal. For instance, if the signal frequency is between 5 Hz and 15 Hz, in particular 10 Hz, the brain wave is of the α type, and if the signal frequency is between 15 Hz and 25 Hz, in particular 20 Hz, the brain wave is of the β type.

In a step S3, the at least one processor determines a psychic state based on the amplitude of at least one brain wave by comparing the amplitude to a predetermined threshold. For instance, a level of attention of the person can be determined based on the comparison.

Optionally, the at least one processor can compute a quality index of the electrical signals detected by the electrodes based on a correlation computation between the common mode measurement and the differential measurement, the expression "differential measurement" referring to the measurement corresponding to the difference between the first electrical signal (i.e. reference signal or measured signal) and the second electrical signal (i.e., measured signal or reference signal) in the single earpiece embodiments and between the measurement signal and the reference signal in the dual earpiece embodiments. Such a correlation computation provides with an estimation of the impedance of the system and therefore of the quality of the measured signal.

There are many applications for the device 1a, 1b, particularly in medicine, such as monitoring patients suffering from neuronal diseases, e.g. epilepsy, screening and diagnosing neuronal diseases, screening and monitoring children suffering from attention disorders, sleep measurements, among others.

## Claims

1. A device (1a, 1b) for determining a physiological or psychological state of a mammal, the device (1a, 1b) comprising at least one earpiece (2,2') comprising:
- a main body (3) comprising an elongation body (31) extending along an elongation axis (32), and
- an earmold (4) configured to be inserted into an ear canal (10, 10') of said mammal, said earmold (4) having a channel (41) adapted to receive said elongation body (31) to removably mount the earmold (4) onto the main body (3), said earmold (4) comprising at least one electrode (42ₖ) arranged on an outer surface of the earmold (4), said at least one electrode (42ₖ) being configured to detect at least one electrical signal in the ear canal (10, 10') of the mammal, said earmold (4) being arranged to be rotatable about the elongation axis (32) so as to allow said at least one electrode (42ₖ) to be directed to an area of the brain of said mammal;
said earpiece (2, 2') comprising at least one first electrical track (43ₖ) arranged in said channel (41) of the earmold (4) and connected to said at least one electrode (42ₖ), and at least one second electrical track (33ₖ) arranged in said elongation body (31) of the main body (3) and configured to convey said at least one electrical signal detected by said at least one electrode (42ₖ) to processing unit (5),
wherein said processing unit (5) comprise at least one preamplifier (6) positioned upstream in the direction of propagation of said at least one electrical signal detected by said at least one electrode (42ₖ), said at least one electrical signal having a given intensity, said at least one preamplifier (6) being configured to amplify said given intensity of at least one electrical signal detected by said at least one electrode (42ₖ).

2. The device (1a, 1b) according to claim 1, wherein the at least one preamplifier (6) is housed in the elongation body (31).

3. The device (1a, 1b) according to any one of the preceding claims, wherein the at least one preamplifier (6) presents a working frequency range extending from 0.1 Hz to 1 kHz.

4. The device (1a, 1b) according to any one of the preceding claims, wherein each among the at least one preamplifier (6) is coupled to an ESD circuit.

5. The device (1a, 1b) according to any one of the preceding claims, wherein the at least one preamplifier comprises a follower circuit.

6. The device (1a, 1b) according to claim 4, wherein the follower circuit is an active circuit.

7. The device (1a, 1b) according to any one of the preceding claims, wherein the at least one preamplifier comprises an impedance matching circuit.

8. The device (1a, 1b) according to any one of the preceding claims, wherein the at least one preamplifier (6) is mounted on a printed circuit board (61) of maximum dimension 3 mm.

9. The device (1a, 1b) according to claim 8, wherein said printed circuit board (61) is made out of a flexible material, so as to be folded.

10. The device (1a, 1b) according to claim 8 and claim 2, wherein said printed circuit board comprises a U-shape so as to embrace additional components embedded in the elongation body (31).

11. The device (1a, 1b) according to any of the preceding claims, wherein the earpiece (2,2') is further configured to transmit a sound signal into the ear canal by means of at least one electroacoustic transducer housed in the elongation body (31), the at least one preamplifier (6) being attached to the at least one electroacoustic transducer along the circumferential direction of the elongation axis (32).

12. The device (1a) according to any of the preceding claims, wherein said at least one electrode (42ₖ) comprises at least a first electrode (42i) being configured to detect a first electrical signal and a second electrode (42₂) configured to detect a second electrical signal,
said at least first electrical track (43ₖ) comprises at least two first electrical tracks, and said at least second electrical track (33ₖ) comprises at least two second electrical tracks,
said first electrode (42i) and second electrode (42₂) being spaced apart in a circumferential direction about the elongation axis (32),
said at least two first electrical tracks being electrically insulated from each other,
said at least two second electrical tracks being electrically insulated from each other, the processing unit (5) being connected to at least one processor,
said at least one processor being configured to determine said physiological or psychological state as a function of said first electrical signal measured by said first electrode (42₁) and of said second electrical signal measured by said second electrode (42₂), in particular as a function of a difference between said first electrical signal and said second electrical signal.

13. The device (1b) according to any one of claims 1 to 7, wherein the device (1) comprises two earpieces (2,2') configured to be arranged respectively in a first ear canal (10) of the mammal and a second ear canal (10') of the mammal and configured to convey respective electrical signals to the processing unit (5),
the processing unit (5) being connected to at least one processor,
said at least one processor being configured to determine said physiological or psychological state as a function of a signal measured by an electrode of the earpiece (2) hosted in the first ear canal (10) and a signal measured by an electrode of the earpiece (2') hosted in the second ear canal (10'), in particular as a function of a difference between said two signals.

14. A computer-implemented method (100) for determining a physiological or psychological state of a mammal by means of at least one processor of a device (1a, 1b) according to claims **12** or **13,** said method comprising:
- determining an electroencephalogram signal of the mammal based on the detected electrical signals,
- determining an amplitude of at least one brain wave in a predefined frequency range as a function of said electroencephalogram signal, and
- determining a psychic state based on said amplitude of at least one brain wave by comparing said amplitude to a predetermined threshold.

15. The method according to claim **14,** further comprising computing a quality index of the electrical signals detected by the electrodes based on a correlation computation between a common mode measurement and said difference between said first electrical signal and said second electrical signal or said difference between said two signals.
